# EUROPEAN PATENT APPLICATION

(11) **EP 4 424 836 A1**
(43) Date of publication of application: **04.09.2024**
(21) Application number: 22886868.3
(22) Date of filing: 20.10.2022
(51) Int. Cl.: C12Q 1/02, C12Q 1/6869, G01N 33/53

(54) **METHOD FOR SELECTING TISSUE-SPECIFIC EXTRACELLULAR VESICLE MARKER, TISSUE-SPECIFIC MARKER, AND METHOD FOR PURIFYING SAME**

(30) Priority: 26.10.2021 JP 2021174911
(71) Applicant: National Institutes of Biomedical Innovation, Health and Nutrition, Ibaraki-shi, Osaka 567-0085 (JP)
(72) Inventor: ADACHI, Jun, Ibaraki-shi, Osaka 567-0085 (JP); MURAOKA, Satoshi, Ibaraki-shi, Osaka 567-0085 (JP); TOMONAGA, Takeshi, Ibaraki-shi, Osaka 567-0085 (JP)
(74) Representative: Dr. Schön, Neymeyr & Partner Patentanwälte mbB
(86) International application number: PCT/JP2022/039192
(87) International publication number: WO 2023/074541

(57) **Abstract**

By isolating high purity plasma and serum extracellular vesicles by affinity capture and comparing them with tissue-specific RNA expression data, proteins that were detected in extracellular vesicles and were found to have tissue-specific expression were selected as tissue-specific extracellular vesicle markers. This has allowed the provision of markers with higher specificity, relative to the tissue-specific markers conventionally reported. Use of the tissue-specific markers allows use thereof in diagnosis with high accuracy. Moreover, use of a marker expressed on the extracellular vesicles allows purification of tissue-specific extracellular vesicles with high purity, and they are therefore also useful for drug discovery research and development.

## Description

### Technical Field

The present invention relates to a method for purifying tissue-specific extracellular vesicles. Specifically, the present invention relates to a method for selecting a marker for purifying extracellular vesicles according to the tissue from which they are derived, and a method for purifying extracellular vesicles using a selected marker. The present invention also relates to a tissue-specific extracellular vesicle marker.

### Background Art

Extracellular vesicles are particles secreted from most tissues and cells, having no nucleus, and enclosed with lipid bilayer membrane. Extracellular vesicles contain proteins, nucleic acids, and lipids that are involved in various physiological processes from derived cells. Therefore, extracellular vesicles derived from body fluids are considered very useful as samples for detecting biomarkers.

Extracellular vesicles are classified, based on the difference in the production mechanism and in the size, as exosomes (50-150 nm), which are secreted out of cells after fusion of multivesicular bodies and the cell membrane; microvesicles (150-1000 nm), which are secreted out of cells by budding directly from the cell membrane; and apoptotic vesicles (1000-5000 nm), which are secreted from apoptotic cells.

Extracellular vesicles, particularly exosomes and microvesicles, are considered to have important roles in cell-cell communication in metastasis of cancer and progression of neurodegenerative diseases and are therefore studied intensively. The diagnosis using extracellular vesicles in blood is noninvasive and therefore imposes less burden on patients even in continuous observation, leading to a higher probability of detecting changes due to disease in the early stage. For example, dementia including Alzheimer disease is difficult to diagnose in the early stage, but if noninvasive diagnosis becomes possible, it is expected to greatly contribute to the development of treatments and therapeutic agents. Moreover, cancer may hopefully be cured if its treatment starts in the early stage, therefore expectations are high for the development of a diagnostic method using extracellular vesicles. Furthermore, extracellular vesicles are considered to serve as a career that intercellularly transports nucleic acid molecules such as miRNA and mRNA and to have an important role in intercellular communication, and expectations are therefore also high for treatment methods using extracellular vesicles or treatment methods targeting extracellular vesicles as a therapeutic target.

Many extracellular vesicles are contained in body fluids and are considered to include changes of the cells from which they are derived, and there are therefore expectations for the development of a diagnosis method using extracellular vesicles. However, extracellular vesicles secreted from various cells are not homogeneous population in size and in properties. Moreover, in the case where extracellular vesicle are classified by size, exosomes and microvesicles are difficult to distinguish one from the other, since they are similar in size and in terms of their components. Moreover, in the case where they are intended to be classified by markers, there has been no marker with a high consensus, and studies have been conducted with vague definitions. As methods for isolating extracellular vesicles, many methods, such as methods involving a combination of centrifugation, methods involving a combination of equilibrium density gradient centrifugation, methods involving separation with a column, and methods involving affinity purification, have been used. Depending on the method of isolating extracellular vesicles, isolated extracellular vesicles are different, and the ratios of contaminants contained are different, and, therefore, a method for purifying extracellular vesicles in high purity and standardization of the method for purification have also been needed.

Moreover, extracellular vesicles in body fluids are a mixture of extracellular vesicles secreted from all types of tissue. Therefore, it is considered that, even if a marker reflecting a disease is contained, the ratio thereof is considered to be very low, and the earlier the disease stage is, the ratio is likely to be lower. If purification of extracellular vesicles according to the tissue from which they are derived is possible, it is considered to increase diagnostic accuracy and to be useful in treatment too. Establishment of a marker for purifying tissue-specific extracellular vesicles, a method for identification thereof, and/or a method for purification thereof will allow us to diagnose diseases with high accuracy.

An attempt to isolate tissue-specific extracellular vesicles and to apply them to diagnosis, etc., has already been disclosed. Patent Literature 1 discloses a method and a device for isolating brain-specific extracellular vesicles, etc., and discloses a method involving isolating brain-specific extracellular vesicles using an antibody that specifically recognizes a protein expressed in the brain, such as tau protein or amyloid beta, and making use thereof in diagnosis, etc.

### Citation List

### Patent Literature

Patent Literature 1:
International Publication No. WO 2015/130956

### Non Patent Literature

Non Patent Literature 1:
   Norman, M. et al., 2021, Nature Methods, Vol.18, pp. 631-634.
Non Patent Literature 2:
   Cao, F. et al., 2019, Electrophoresis Vol.40, pp. 3092-3098.
Non Patent Literature 3:
   Palviainen, M. et al., 2020, PLOS ONE 15 (8), e0236439.
Non Patent Literature 4:
   Pietrowska, M. et al., 2021, J. Extracell. Vesicles 10 (4), e12063.
Non Patent Literature 5:
   Ding, X.-Q. et al., 2020, Front Oncol., doi.org/10.3389/fonc.2020.01113
Non Patent Literature 6:
   Jeannin, P. et al., 2018, Sci. Rep. Vol. 8: 5170.
Non Patent Literature 7:
   Fel, A. et al., 2019, Proteomes, doi.org/10.3390/proteomes7020020.

### Summary of Invention

### Technical Problem

The invention according to Patent Literature 1 is a method for isolating extracellular vesicles secreted from the brain using a protein found to be expressed in the brain. However, tau protein, for example, is expressed mainly in the central nervous system such as the brain, but is also expressed in peripheral nerves and glia cells. Moreover, amyloid beta is also found to be expressed in platelets, blood vessels, muscles, etc., and thus cannot necessarily be said to be specifically expressed in the brain. Therefore, extracellular vesicles derived from the brain are concentrated, but a certain amount of extracellular vesicles secreted from other tissues is also contained.

Furthermore, it has been shown that L1CAM, which has conventionally been used as a marker for extracellular vesicles derived from the brain, is not contained in the fractions of extracellular vesicles in plasma and in the cerebrospinal fluid, and is not available for isolation of nervous tissue-specific extracellular vesicles (Non Patent Literature 1). Some proteins are used as tissue-specific extracellular vesicle markers only because they are proteins highly expressed in specific tissues, but such proteins may include those not sufficiently appropriate as a marker.

As described above, extracellular vesicles are a population of vesicles secreted from respective tissues, and neither methods for identifying a marker for purification according to the tissue from which they are derived nor methods for purification thereof have been established yet. As indicated in Non Patent Literature 1, a protein found to be expressed in a tissue-specific manner is not necessarily contained in extracellular vesicles. Moreover, the tissue-specific extracellular vesicle markers currently being used are expressed not only in a particular tissue, but are often found to be expressed in a plurality of tissues. Therefore, it is expected that purification of extracellular vesicles with a conventional "tissue-specific extracellular vesicle marker" would not necessarily result in a high degree of purification.

Moreover, it has been also reported that there is a difference in molecular composition between plasma and serum extracellular vesicles (Non Patent Literature 2, 3). However, since samples are blood samples under current situations, extracellular vesicles contained in plasma and serum are considered almost the same, and also tissue-specific markers have not been examined for plasma and serum, separately.

An object of the present invention is to provide a method for selecting a marker for isolating tissue-specific extracellular vesicles. Furthermore, an object of the present invention is to provide a method involving searching for tissue-specific extracellular vesicle markers, and purifying tissue-specific extracellular vesicles using the same. The marker selected by the method for selection according to the present invention is a marker with higher tissue specificity, and extracellular vesicles purified using the marker will therefore be those with higher tissue specificity. It is considered that obtaining extracellular vesicles with high tissue specificity would contribute to diagnosis and/or drug discovery research and development using extracellular vesicles.

### Solution to Problem

The present invention relates to a tissue-specific extracellular vesicle marker described below and to a method for purifying tissue-specific extracellular vesicles using the same. The present invention also relates to a method for selecting a tissue-specific extracellular vesicle marker.
(1) A method for purifying brain tissue-specific extracellular vesicles, comprising: purifying plasma extracellular vesicles or serum extracellular vesicles; and purifying brain tissue-specific extracellular vesicles using a specific reagent that binds to at least one marker selected, when using plasma extracellular vesicles, from GPM6A, LLGL1, CNP, MBP, and GNG7, which are detected in plasma extracellular vesicles, or selected, when using serum extracellular vesicles, from GDI1 and SLC44A1, which are detected in serum extracellular vesicles.
(2) A brain tissue-specific marker, wherein the marker is at least one selected from GPM6A, YWHAH, LLGL1, CNP, STXBP1, MBP, and GNG7, which are detected in plasma extracellular vesicles, and GDI1, YWHAH, and SLC44A1, which are detected in serum extracellular vesicles.
(3) A method for purifying liver tissue-specific extracellular vesicles, comprising, purifying plasma extracellular vesicles or serum extracellular vesicles; and using a specific reagent that binds to at least one marker selected, when using plasma extracellular vesicles, from ABCB11, CYP4F3, DCXR, SLC38A3, TFR2, SLC27A5, and ABCB4, which are detected in plasma extracellular vesicles, or selected, when using serum extracellular vesicles, from SLC27A2, CYP4F3, DCXR, SLC38A3, TFR2, SLC27A5, and ABCB4, which are detected in serum extracellular vesicles.
(4) A liver tissue-specific marker, wherein the marker is at least one selected from ABCB11, CYP4F3, DCXR, SLC38A3, TFR2, SLC27A5, ABCB4, UGDH, ARG1, ADH4, MTHFD1, AKR1D1, and GRHPR, which are detected in plasma extracellular vesicles, and SLC27A2, CYP4F3, DCXR, SLC38A3, TFR2, SLC27A5, ABCB4, ARG1, ADH4, MTHFD1, AKR1D1, RAB43, HGD, and GRHPR, which are detected in serum extracellular vesicles.
(5) A standard protein for plasma-derived extracellular vesicles, wherein the protein is at least one selected from NCK1 and ACTBL2.
(6) A standard protein for serum-derived extracellular vesicles, wherein the protein is at least one selected from NCK1 and ARF6.
(7) A method for selecting a standard protein for extracellular vesicles, comprising: analyzing extracellular vesicles from a plurality of healthy individuals; calculating coefficients of variation for detected proteins; and selecting a protein with a small coefficient of variation as the standard protein.
(8) A method for selecting a tissue-specific marker in liquid biopsy, comprising: purifying extracellular vesicles from a body fluid; and, among proteins exhibiting tissue-specific expression in RNA sequence data showing information on human protein expression, selecting a protein detected in an extracellular vesicle fraction as a marker to be used for selection of tissue-specific extracellular vesicles.
(9) The method for selecting a tissue-specific marker according to (8), wherein the proteins exhibiting tissue-specific expression in RNA sequence data are proteins found to exhibit at least 4 times change in the tissue of interest in comparison with other tissues.
(10) The method for selecting a tissue-specific marker according to (8) or (9), wherein the RNA sequence data showing information on human protein expression is RNA sequence analysis data in Human Protein Atlas.
(11) The method for selecting a tissue-specific marker according to any one of (8) to (10), wherein the method for purifying extracellular vesicles is affinity capture.
(12) The method for selecting a tissue-specific marker according to any one of (8) to (11), wherein the body fluid is blood, and the extracellular vesicles are purified from plasma and/or serum.
(13) A lymphoid-specific marker, wherein the marker is at least one selected from GRAP2, CD3E, and CD3G, which are detected in plasma extracellular vesicles.
(14) A skeletal muscle-specific marker, wherein the marker is at least one selected from STK25, ALDOA, PKM, PGM1, and CHMP1B, which are detected in serum extracellular vesicles.
(15) A bone marrow-specific marker, wherein the marker is at least one selected from HBD, SPTA1, HBB, and HBA1, which are detected in serum extracellular vesicles.
(16) A liver-specific marker, wherein the marker is at least one selected from UGDH, ABCB11, ARG1, ADH4, MTHFD1, CYP4F3, DCXR, SLC38A3, GRHPR, and SLC27A5, which are detected in plasma extracellular vesicles.
(17) A liver-specific marker, wherein the marker is at least one selected from SLC27A2, ARG1, ADH4, AKR1D1, CYP4F3, RAB43, HGD, SLC38A3, TFR2, and SLC27A5, which are detected in serum extracellular vesicles.
(18) A method for purifying bone marrow tissue-specific extracellular vesicles using a specific reagent that binds to at least one marker selected from GYPA, MPO, CTSG, and RHAG, which are detected in plasma and serum extracellular vesicles, and TSPO2, which is detected in plasma extracellular vesicles.
(19) A method for purifying brain tissue-specific extracellular vesicles using a specific reagent that binds to at least one marker selected from GNG7, ENO2, ATP1B2, RAB3A, RPKAR1B, GPM6A, KLC1, TIAM1, LLGL1, RTN1, SLC44A1, STMN2, LRRC7, RSD2, TTYH2, NAPB, TMEM59L, CTNND2, MAST1, and SLC6A5, which are detected in plasma and serum extracellular vesicles, SLIT1 and SLC26A5, which are detected in plasma extracellular vesicles, and HTR2A and MTCL1, which are detected in serum extracellular vesicles.
(20) A method for purifying breast tissue-specific extracellular vesicles using a specific reagent that binds to ABCC11, which is detected in plasma extracellular vesicles.
(21) A method for purifying myocardial tissue-specific extracellular vesicles using a specific reagent that binds to PKP2, which is detected in plasma and serum extracellular vesicles.
(22) A method for purifying intestinal tissue-specific extracellular vesicles using a specific reagent that binds to at least one marker selected from MGAM, TSPAN8, PLCB3, GPA33, and ACSL5, which are detected in plasma and serum extracellular vesicles, CDH17, ENTPD8, and CDHR2, which are detected in plasma extracellular vesicles, and VIL1, which is detected in serum extracellular vesicles.
(23) A method for purifying kidney tissue-specific extracellular vesicles using a specific reagent that binds to at least one marker selected from PDZK1IP1, SLCO4C1, and TRPV5, which are detected in plasma and serum extracellular vesicles.
(24) A method for purifying lung tissue-specific extracellular vesicles using a specific reagent that binds to at least one marker selected from SFTPB, which is detected in plasma and serum extracellular vesicles, and SCGB3A2, which is detected in plasma extracellular vesicles.
(25) A method for purifying lymphoid tissue-specific extracellular vesicles using a specific reagent that binds to at least one marker selected from CTSV, CD1A, GP1BA, CD3E, CD3G, CR2, KCNA3, and PSD4, which are detected in plasma and serum extracellular vesicles.
(26) A method for purifying pancreas tissue-specific extracellular vesicles using a specific reagent that binds to at least one marker selected from PRSS3 and SLC38A5, which are detected in plasma and serum extracellular vesicles.
(27) A method for purifying parathyroid tissue-specific extracellular vesicles using a specific reagent that binds to at least one marker selected from ACSL3, CD109, SLC17A5, and SLC7A8, which are detected in plasma and serum extracellular vesicles.
(28) A method for purifying placenta tissue-specific extracellular vesicles using a specific reagent that binds to at least one marker selected from MAN1A2, SLC2A1, LUM, and LNPEP, which are detected in plasma and serum extracellular vesicles.
(29) A method for purifying retina tissue-specific extracellular vesicles using a specific reagent that binds to KCNV2, which is detected in plasma and serum extracellular vesicles.
(30) A method for purifying salivary gland tissue-specific extracellular vesicles using a specific reagent that binds to at least one marker selected from PRH1 and ZG16B, which are detected in plasma and serum extracellular vesicles, and CST4, SMR3B, and MUC7, which are detected in plasma extracellular vesicles.
(31) A method for purifying skeletal muscle tissue-specific extracellular vesicles using a specific reagent that binds to at least one marker selected from XIRP2 and SHISA4, which are detected in plasma and serum extracellular vesicles.
(32) A method for purifying skin tissue-specific extracellular vesicles using a specific reagent that binds to at least one marker selected from DSC1, CST6, ASPRV1, SERPINA12, and WFDC5, which are detected in plasma and serum extracellular vesicles.
(33) A method for purifying testis tissue-specific extracellular vesicles using a specific reagent that binds to at least one marker selected from GORASP2, which is detected in plasma and serum extracellular vesicles, SLC2A14, which is detected in plasma extracellular vesicles, and CTAGE1 and PRSS50, which are detected in serum extracellular vesicles.
(34) A method for purifying thyroid gland tissue-specific extracellular vesicles using a specific reagent that binds to PKHD1L1, which is detected in plasma and serum extracellular vesicles.
(35) A method for purifying tongue tissue-specific extracellular vesicles using a specific reagent that binds to at least one marker selected from LCN1, which is detected in plasma and serum extracellular vesicles, and VSIG8, which is detected in plasma extracellular vesicles.

Conventionally, proteins highly expressed in particular tissues have been used for purifying tissue-specific extracellular vesicles, having been considered that they are also found in extracellular vesicles. However, the use of markers selected using the method according to the present invention has made it possible to concentrate and isolate extracellular vesicles with higher tissue specificity.

### Brief Description of Drawings

[Figure 1] Figure 1 shows a method for isolating extracellular vesicles from plasma and serum by affinity capture, and properties of isolated extracellular vesicles.
FIG.1A schematically shows the method for purifying extracellular vesicles.
FIG.1B shows properties of extracellular vesicles isolated from plasma and serum by nanoparticle tracking analysis.
FIG.1C shows properties of extracellular vesicles isolated from plasma and serum by transmission electron micrographs.
FIG.1D shows properties of extracellular vesicles isolated from plasma and serum by expression analysis of extracellular vesicle markers by Western blotting.
[Figure 2] Figure 2 shows protein profiling of extracellular vesicles isolated from plasma or serum.
FIG.2A shows the number of proteins identified in extracellular vesicles obtained from plasma and serum fractions.
FIG.2B shows a result of gene ontology analysis by DAVID v6.8.
FIG.2C shows the comparison of the number of marker proteins and contaminated proteins identified in extracellular vesicles (upper) and the ratio of the identified proteins (lower) in the method according to the present invention and the data published in 4 previous studies.
[Figure 3] Figure 3 shows a result of comparative analysis of proteins in extracellular vesicles isolated from plasma or serum obtained from 6 healthy individuals.
FIG.3A shows a result of principal component analysis of extracellular vesicle proteins derived from plasma and serum.
FIG.3B is a scatter plot showing the correlation between the expression levels of plasma and serum extracellular vesicle proteins.
FIG.3C shows a histogram of coefficients of variation among samples for plasma and serum extracellular vesicle proteins.
FIG.3D shows the expression levels of proteins with small coefficients of variation detected in plasma, serum, and serum and plasma in extracellular vesicle samples of 6 healthy individuals.
[Figure 4] Figure 4 shows tissue-specific proteins contained in plasma and serum extracellular vesicles.
FIG.4A shows the number of tissue-specific proteins contained in plasma and serum extracellular vesicles.
FIG.4B shows proteins having positive correlation in expression between plasma and serum brain tissue-specific extracellular vesicles.
FIG.4C shows interaction between proteins identified as brain tissue-specific molecules by Ingenuity Pathway Analysis in plasma and serum extracellular vesicles.

### Description of Embodiments

The present invention relates to a method for selecting tissue-specific extracellular vesicles. A method for selecting brain, liver, lymph, skeletal muscle, or bone marrow-specific extracellular vesicles is herein described as an embodiment, but it is needless to say that the application of the method is not limited to these tissues and the method can be applied to any tissue.

A method using serum and plasma is herein described as an example because serum and plasma are less invasive and excellent in continuous observation and because they are common samples also used in other examinations, but it is needless to say that body fluid samples other than serum and plasma may be used. By similar examinations, tissue-specific extracellular vesicle markers can also be selected for samples that are collected in a noninvasive or low-invasive manner, such as biological samples other than the blood samples, for example, urine, cerebrospinal fluid, saliva, lymph fluid, lacrimal fluid, pleural fluid, or ascites.

Moreover, tissue-specific extracellular vesicle markers described below may be used alone or in combination. Such a tissue-specific extracellular vesicle may be isolated and/or purified using a specific reagent that recognizes the marker, such as an aptamer or an antibody, in specific, and used in diagnosis, etc., after the isolation of extracellular vesicles, if the tissue-specific marker is expressed on the surface of extracellular vesicles.

### [Isolation of extracellular vesicles from plasma and serum samples]

Blood samples are widely used because they are less invasive among the samples used in liquid biopsy. However, it was reported that, as stated above, plasma and serum provide different results when used in the examination of extracellular vesicles. Therefore, properties of extracellular vesicles isolated from plasma and serum were examined.

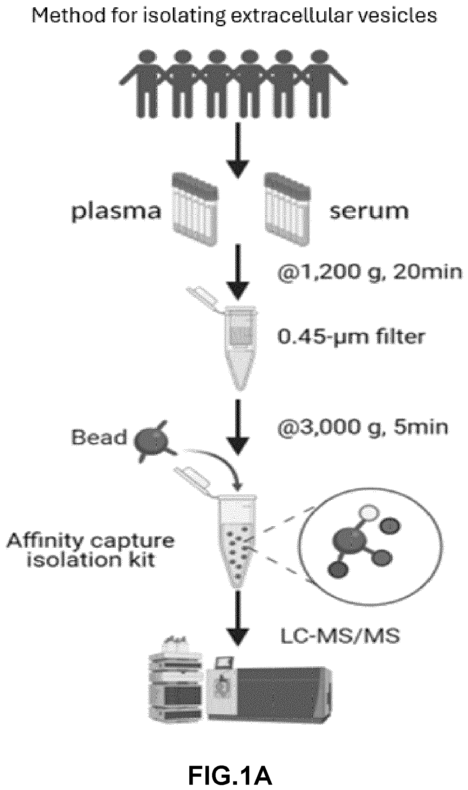

From 6 healthy individuals, blood was collected; plasma samples were prepared by adding EDTA, and serum samples were prepared without adding any anticoagulant. To each 150 µl of the plasma and the serum, 350 µl of tris-buffered saline was added, and the mixture was centrifuged at 1200g for 20 min. at 4°C. The supernatant was filtered with a centrifuge tube filter with 0.45 µm pore size (Corning), and 5 µl of Heparin Sodium Solution (1 U/µl, FUJIFILM Wako Pure Chemical Corporation) was added to the EDTA plasma samples for maintaining anticoagulant effect. From the obtained samples, extracellular vesicles were captured using MagCapture Exosome Isolation Kit PS Ver.2 (FUJIFILM Wako Pure Chemical Corporation). The extracellular vesicles were eluted with 100 µl of an eluent and 80 µl was used for proteomics analysis (FIG.1A). This affinity capture is affinity purification using beads on which Tim4 (T-cell immunoglobulin domain and mucin domain-containing protein 4) protein, which binds in a calcium ion-dependent manner to phosphatidylserine exposed on the outside of the membrane of extracellular vesicles, is immobilized. Alternatively, extracellular vesicles may be purified by affinity capture using an antibody that recognizes a surface antigen of extracellular vesicles, such as CD9, CD63, or CD81, or Annexin A5, which is known to bind to phosphatidylserine, but As described later, it is preferable to use highly purified extracellular vesicles with a small proportion of non-extracellular vesicle proteins.

For proteomics analysis of isolated extracellular vesicles, the nano LC-MS/MS analysis was conducted with LTQ-Orbitrap Fusion Lumos massspectrometer (Thermo Fisher Scientific) equipped with UltiMate 3000 Nano LC system (Thermo Fisher Scientific) and HTC-PAL autosampler (CTC Analysis).

### [Properties of extracellular vesicles isolated from plasma and serum]

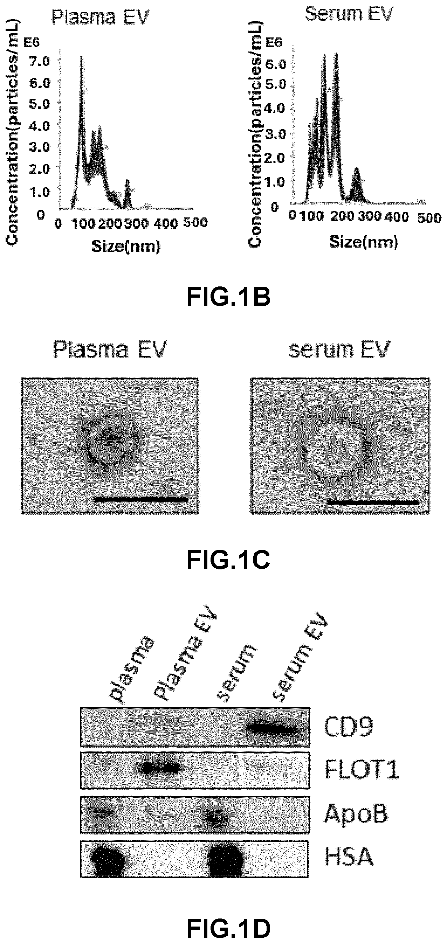

FIG.1B shows a result of the nanoparticle tracking analysis of extracellular vesicles obtained from plasma and serum. Extracellular vesicles isolated from plasma is referred to as plasma extracellular vesicles, and extracellular vesicles isolated from serum is referred to as serum extracellular vesicles. Moreover, extracellular vesicles may be denoted as EV (Extracellular Vesicle) in tables, drawings, etc.

For the nanoparticle tracking analysis, 30 second video image of each sample was acquired five times at 21°C with Nanosight 300 (Malvern Panalytical Ltd) and analyzed with the NTA3.3 software (Malvern Panalytical Ltd). Extracellular vesicles obtained by affinity capture are 100-300 nm in size and are considered to contain exosomes and microvesicles. The numbers of particles and the amounts of protein in each sample before and after purification are shown in Table 1. It was shown that proteins contained as contaminants were removed from plasma or serum, and extracellular vesicles were purified by affinity capture.

**[Table 1]**

| | plasma | Plasma EV fraction | serum | Serum EV fraction |
|---|---|---|---|---|
| Particle number | 2.70E+11 | 1.17E+09 | 5.70E+11 | 1.28E+09 |
| EV protein (µg) | 11400 | 0.898 | 11400 | 0.84 |
| Particles/proteins(µg) | 2.37E+07 | 1.31 E+09 | 5.00E+07 | 1.50E+09 |

In the table, the number of particles in plasma and serum indicates the number of particles in 150 µl used for purification and the number of particles in the plasma EV fractions and the serum EV fractions indicates the number of particles in 100 µl, which is the volume eluted by affinity capture.

The extracellular vesicles isolated from either plasma or serum exhibited morphologies similar to those reported for exosomes when they were negatively stained with uranium acetate and observed with a transmission electron microscopy (Hitachi High-Tech Corporation, H-7600) (FIG.1C). Furthermore, CD9 and flotillin (FLOT1), which are said to be extracellular vesicle markers, were detected in the extracellular vesicles isolated from either plasma or serum when examined by the Western blotting in a common procedure. In contrast, none of apolipoprotein B (ApoB) and human serum albumin (HSA), which were described as contaminant markers in the guideline (MISEV2018) provided by the International Society for Extracellular Vesicles (ISEV) in 2018, was detected in the serum extracellular vesicle fraction, while apolipoprotein B was slightly detected in the plasma extracellular vesicle fraction (FIG.1D). The detection was conducted using Anti-CD9 antibody (D3H4P, Cell Signaling Technology), Anti-flotillin antibody (#EPR6041, Abeam), Anti-apolipoprotein B antibody (A-6, Santa Cruz Biotechnology), or Anti-human serum albumin (HSA) antibody (M41131455, R & D systems) as a primary antibody and HRP-labeled anti-mouse IgG antibody (#7076S, Cell Signaling Technology) or Anti-rabbit IgG antibody (#7074S, Cell Signaling Technology) as a secondary antibody.

### [Profiling of plasma extracellular vesicles and serum extracellular vesicles]

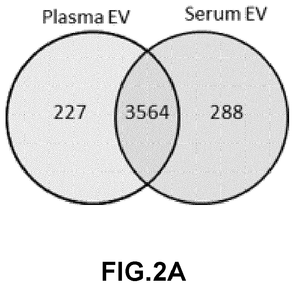

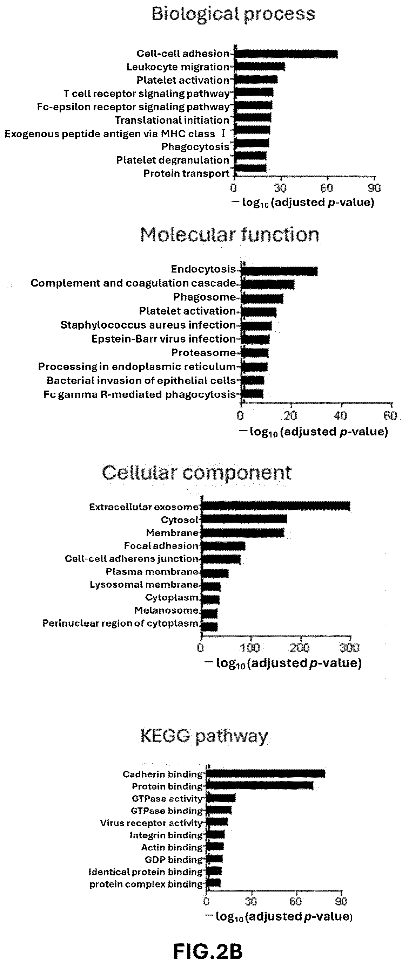

In the extracellular vesicles isolated by the method described above, 4079 proteins in total were detected: among them, 3564 proteins were proteins detected both in plasma and in serum, 227 proteins were proteins detected only in plasma extracellular vesicles, and 288 proteins were proteins detected only in serum extracellular vesicles (FIG.2A). Furthermore, as a result of the gene ontology analysis by DAVID (Database for Annotation, Visualization, and Integrated Discovery), it was revealed that the plasma and serum extracellular vesicles detected by this method contain extracellular exosomes in "Cellular component", and proteins related to cadherin binding and protein binding, GTPase, and GTP binding proteins in "KEGG pathway" in large number. Furthermore, it was shown that they contain proteins involved in leukocyte migration, T cell receptor signaling pathway, endocytosis, and phagosome, which are related to immune functions, in "Biological process" and "Molecular function" in abundance (FIG.2B).

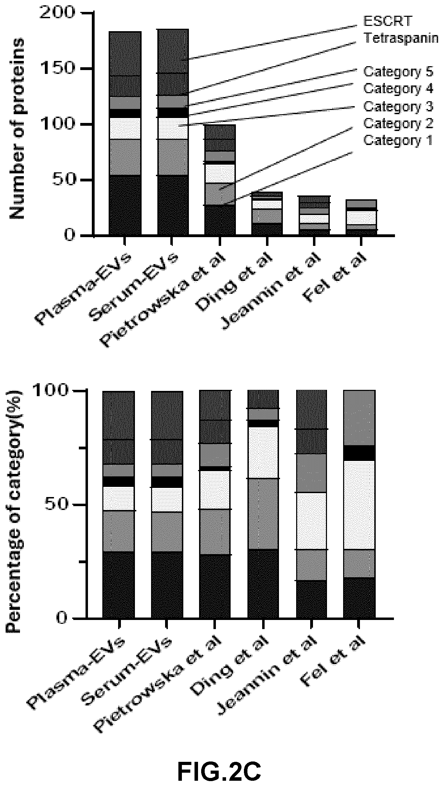

FIG.2C shows the number and the proportion of molecules related to extracellular vesicles described in MISEV 2018 and non-extracellular vesicle molecules in comparison between the extracellular vesicles isolated by this method and those of the 4 previous studies (Non Patent Literatures 4-7). In the 4 previous studies, extracellular vesicles were purified using size exclusion chromatography (Pietrowska et al., Non Patent Literature 4), ultracentrifugation (Ding et al., Non Patent Literature 5), qEV column (IZON Science, Jeannin et al., Non Patent Literature 6), Total Exosome Isolation kit (Invitrogen, Fel et al., Non Patent Literature 7). It was found that, in comparison with the proteins detected in the extracellular vesicle fraction in the 4 previous studies, those in this method contain more proteins in Category 1 (GPI-anchored membrane proteins associated with cell membrane and endosomes), Category 2 (cytosolic proteins collected from extracellular vesicles), the Tetraspanin family, and the ESCRT family. In contrast, the proportion of non-extracellular vesicle proteins classified as Category 3 (non-extracellular vesicle constituents contained in extracellular vesicle fraction) was smaller than that of the 4 previous studies. Thus, the extracellular vesicles isolated in this method contain molecules other than extracellular vesicles less than those isolated in other methods for isolation and are considered as those of a higher degree of purification.

Affinity capture method not only has an advantage of being capable of providing high purity of extracellular vesicles, but also has an advantage of being capable of purifying extracellular vesicles in a shorter time, in comparison with ultracentrifugation etc. To obtain tissue-specific extracellular vesicles, obtaining high purity of extracellular vesicles is very important, and, also in the case of purifying those by other methods, similar purity of extracellular vesicles is required. Moreover, examination with clear distinction of plasma and serum extracellular vesicles is required because, as shown in FIG.2A, different extracellular vesicle proteins are detected in plasma and serum extracellular vesicles, while some proteins are detected in both.

### [Comparative analysis of plasma extracellular vesicle and serum extracellular vesicle proteins]

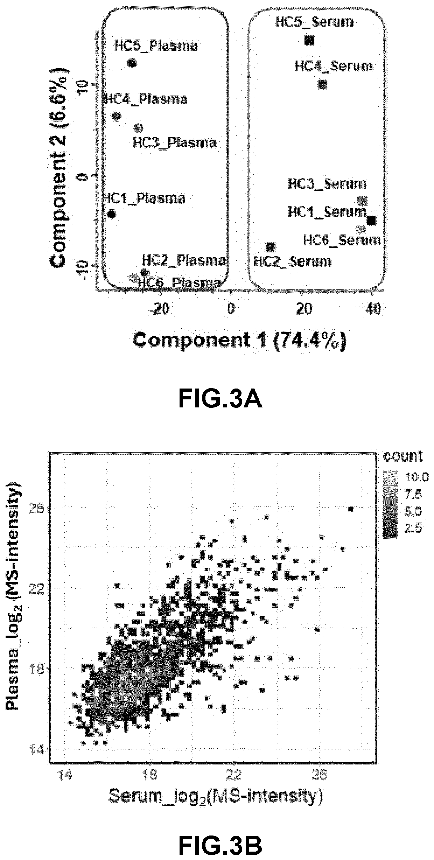

Examination of the proteins contained in the plasma and serum extracellular vesicles was conducted. A principal component analysis indicated slight separation of the plasma and serum extracellular vesicles (FIG.3A). Fig.3B is a scatter plot showing the correlation of expression levels of 1547 proteins commonly detected in the samples of the 6 healthy individuals in the plasma and serum extracellular vesicles. While the expression levels of the proteins contained in the plasma and serum extracellular vesicles indicate a positive correlation (r = 0.7381, p < 0.0001), the expression levels of many proteins were different. The functional analysis of 1547 proteins revealed that, in the plasma extracellular vesicles, more proteins related to complement and coagulation cascade and cholesterol metabolism are detected, relative to those in the serum extracellular vesicles, and, in contrast, hardly any proteins related to platelet activation are contained.

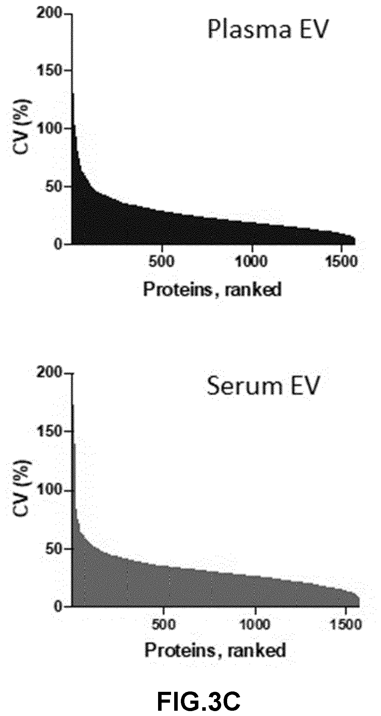

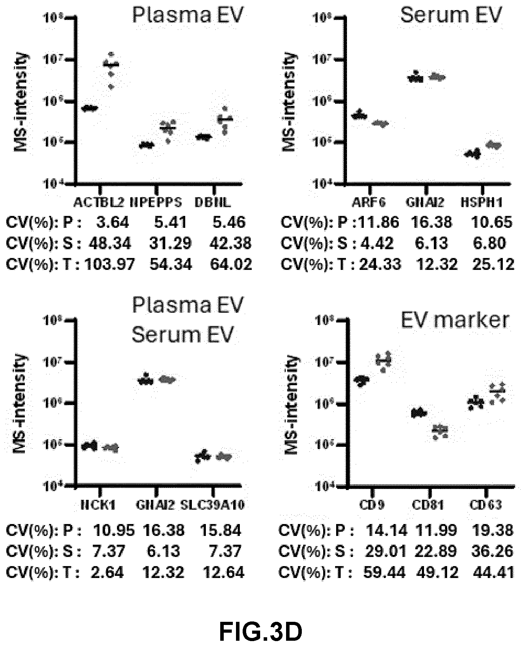

The coefficient of variation was calculated for the plasma and serum proteins in all subjects (FIG.3C). Among the proteins detected in the plasma extracellular vesicles and the serum extracellular vesicles, those with a small coefficient of variation are shown in Table 2 (proteins with a small coefficient of variation in the plasma extracellular vesicles) and Table 3 (proteins with a small coefficient of variation in the serum extracellular vesicles). NCK1, which is a cytoplasm protein, has a very small coefficient of variation (CV) of 10.95 in plasma and 8.70 in serum. As a marker for plasma extracellular vesicles, ACTBL2 (beta-actin like protein, CV = 3.64) has a small coefficient of variation. As a marker for serum extracellular vesicles, ARF6 (ADP-ribosylation Factor 6, CV = 4.42) has a small coefficient of variation. Therefore, they may be used as standard proteins (FIG.3D). Moreover, the proteins listed in Table 2 and Table 3 are proteins with small variation contained in plasma or serum extracellular vesicles and these proteins may therefore be used as standard proteins. Here, examination was conducted with samples from 6 healthy individuals, and a standard protein for any tissue can be selected by a similar method by analyzing samples from at least 5 or more individuals. This is because, in estimation of the probability that a certain tissue-specific protein shows the same tendency of change among individuals, the probability that samples from 5 or more individuals show the same tendency of change by chance is considered very low, provided that the abundance of a protein usually varies among individuals.

### [Proteins with small coefficient of variation in plasma extracellular vesicles]

**[Table 2]**

| Uniprot ID | Genes | CV (%) in plasma EV | CV (%) in serum EV |
|---|---|---|---|
| Q562R1 | ACTBL2 | 3.64 | 48.34 |
| P55786 | NPEPPS | 5.41 | 31.29 |
| Q9UJU6 | DBNL | 5.46 | 42.38 |
| P11766 | ADH5 | 5.64 | 30.80 |
| Q70IA8 | MOB3C | 5.66 | 9.97 |
| P23526 | AHCY | 5.66 | 21.91 |
| P10075 | GLI4 | 5.76 | 34.21 |
| P43034 | PAFAH1B1 | 5.88 | 32.33 |
| P35579 | MYH9 | 5.92 | 35.31 |
| P60660 | MYL6 | 5.95 | 34.30 |

### [Proteins with small coefficient of variation in serum extracellular vesicles]

**[Table 3]**

| Uniprot ID | Genes | CV (%) in plasma EV | CV (%) in serum EV |
|---|---|---|---|
| P62330 | ARF6 | 11.86 | 4.42 |
| P04899 | GNAI2 | 16.38 | 6.13 |
| Q92598 | HSPH1 | 10.65 | 6.80 |
| Q9ULF5 | SLC39A10 | 15.84 | 7.37 |
| Q9BS40 | LXN | 11.63 | 8.11 |
| Q9NUQ9 | FAM49B | 18.07 | 8.35 |
| P02766 | TTR | 47.42 | 8.46 |
| P16333 | NCK1 | 10.95 | 8.70 |
| Q969P0 | IGSF8 | 9.17 | 8.91 |
| P43250 | GRK6 | 16.38 | 9.14 |

CD9, CD81, and CD63, which are widely used as exosome markers, exhibited high CV values in the plasma and serum (FIG. 3D). For the 6 healthy individuals, plasma and serum from the same individual were purified and analyzed 3 times, and the Spearman correlation coefficient and the p value were calculated for 1579 molecules that were quantifiable in all samples and analyzed, revealing that there is difference in the expression pattern of the protein molecules in plasma extracellular vesicles and serum extracellular vesicles (data not shown here). CD9, CD81, and CD63, etc. have conventionally been used as markers for extracellular vesicles, but no consensus has been achieved among researchers. These so-called extracellular vesicle markers, which have conventionally been used, also show variations in the abundance, depending on the method of purifying extracellular vesicles, etc., and are therefore considered as those on which no consensus has been obtained as extracellular vesicle markers. The proteins with a low CV value described herein may be used as standard proteins for indicating the presence and the purity of extracellular vesicles in analysis of plasma and serum extracellular vesicles. Accordingly, antibodies and aptamers that recognize the standard proteins and reagents for the detection thereof may constitute a kit for indicating the presence and the purity of extracellular vesicles.

### [Tissue-specific extracellular vesicles]

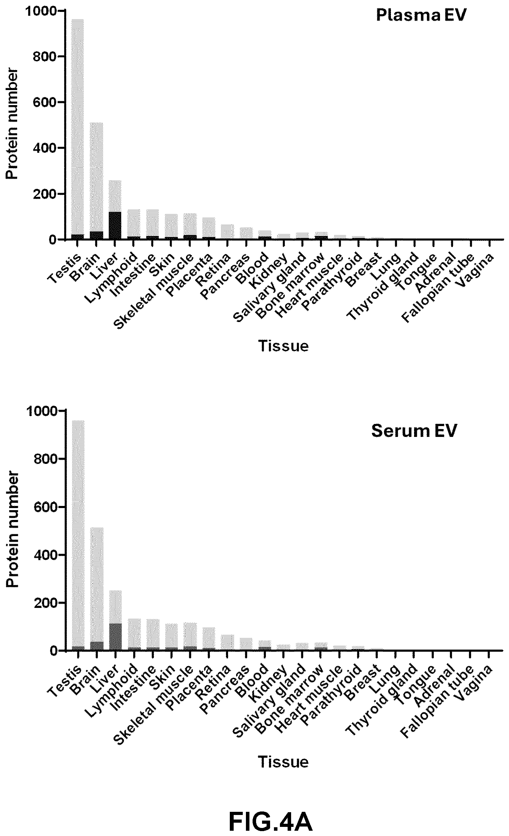

Next, examination on the detection of tissue-specific extracellular vesicles was conducted. Using the RNA sequence analysis data in Human protein atlas (HPA) as a standard, tissue-specific molecules contained in plasma and serum extracellular vesicles were analyzed. Molecules with tissue-specific expression were determined with a condition that they are found to have at least 4 times expression in the tissue of interest, relative to the expression in other tissues, in the data in HPA. Proteins contained in plasma and serum extracellular vesicle fractions were screened for proteins expressed specifically in tissues. FIG.4A shows the number of proteins contained in plasma or serum extracellular vesicles, among the proteins detected in the tissues. In FIG.4A, the data in gray is the data in HPA, and the proteins in dark color are proteins detected in the extracellular vesicles obtained by the method according to the invention.

In the HPA data base, 242 molecules were found as liver-specific molecules: among them, 120 molecules were found in plasma extracellular vesicles, and 112 molecules were found in serum extracellular vesicles. A KEGG pathway analysis revealed that the liver-specific proteins detected in the extracellular vesicles include proteins involved in complement and coagulation cascade and PPAR signaling pathway in large number. This result suggests that blood proteins such as complements and albumin are mainly produced in the liver and are detected as contaminants in the extracellular vesicle fraction. Cytochrome P450 family 4 subfamily F member 3 (CYP4F3), which is related to the drug metabolism, and liver-specific molecules were detected more in the plasma extracellular vesicles than in the serum extracellular vesicles. This result suggests that use of plasma extracellular vesicles is more desirable for the evaluation of drug metabolism in blood. Purification of extracellular vesicles derived from the liver using a liver tissue-specific extracellular vesicle marker present on the membrane, described below, will allow quantitative determination of drug metabolizing enzymes and analysis of the pharmacokinetics.

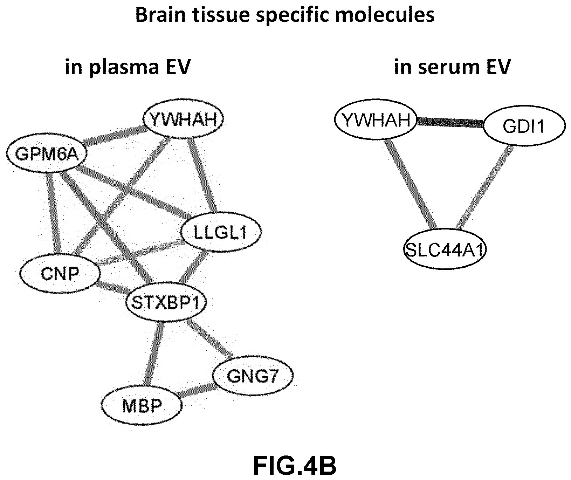

Moreover, it was revealed that the plasma extracellular vesicles contain 34 brain tissue-specific proteins, and the serum extracellular vesicles contain 36 brain tissue-specific proteins (FIG.4A). A covariation analysis revealed that 7 proteins in the plasma extracellular vesicles showed strong correlation, and 3 proteins in the serum extracellular vesicles showed strong correlation (p < 0.001, both in the plasma extracellular vesicles and in the serum extracellular vesicles) (FIG.4B). Moreover, among these proteins, STXBP1, GPM6A, and GDI1 are neuron-specific proteins, and SLC44A1 and CNP are oligodendrocyte-specific proteins.

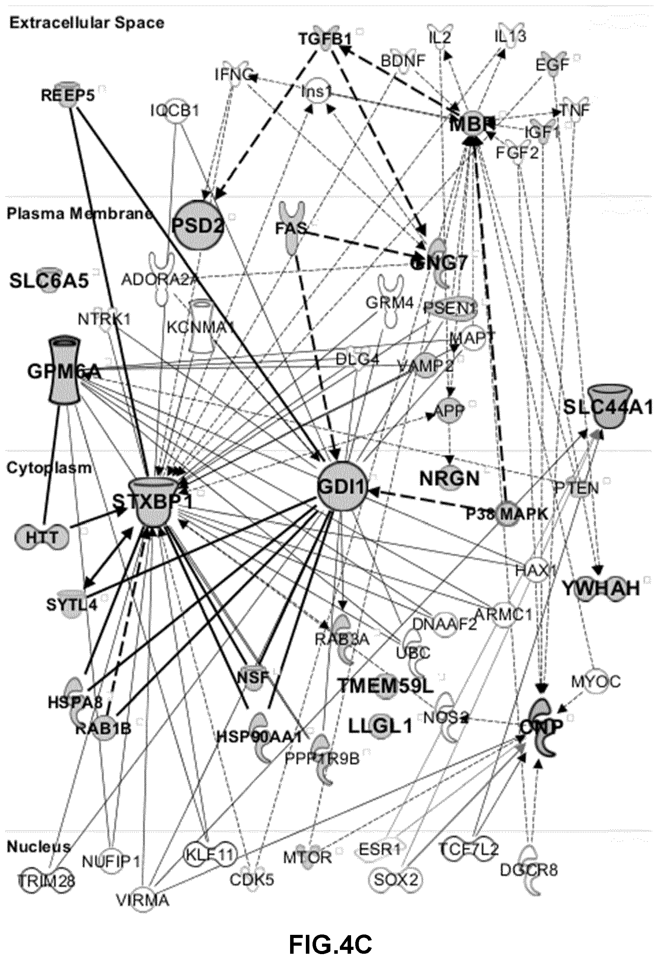

FIG.4C shows direct or indirect interaction, analyzed by Ingenuity Pathway Analysis (QIAGEN), among the 13 proteins (STXBP1, GPM6A, PSD2, GDI1, SLC44A1, CNP, MBP, GNG7, SLC6A5, NRGN, YWHAH, TMEM59L, LLGL) detected as brain tissue-specific proteins in the plasma extracellular vesicles and in the serum extracellular vesicles. GPMA6, STXBP1, and GDI1 indirectly interact with one another via amyloid precursor protein (APP), microtubule-associated protein tau (MAPT), presenilin-1 (PSEN1), or huntingtin (HTT), and are said to be related to neurodegenerative diseases such as Alzheimer disease, progressive supranuclear palsy (PSP), and Huntington's disease (HD). PSEN1, MAPT, APP, and HTT are considered to be contained in the extracellular vesicles derived from the nerve and are considered to be secreted with GPM6A, STXBP1, and GDI1 into the blood through the blood brain barrier from the brain tissue, based on a protein-protein interaction analysis.

Moreover, among the aforementioned brain-specific proteins contained in the extracellular vesicles, YWHAH and STXBP1 are proteins located in cytoplasm and are not proteins expressed on the surface of extracellular vesicles. Therefore, they cannot be used for the purification, but other proteins can be used to purify brain-specific extracellular vesicles. Specifically, this may be achieved by affinity purification using an antibody or an aptamer that recognizes one of the aforementioned proteins present in brain-specific extracellular vesicles.

Furthermore, there is a possibility that analysis of the change of these brain tissue-specific markers in patients with neurodegenerative diseases such as Alzheimer's disease, progressive supranuclear palsy, and Huntington's disease allows the detection of markers specific to these diseases. Identification of a disease-specific marker would allow the marker to be used in the diagnosis, monitoring of the disease or the like.

The extracellular vesicles derived from tissues other than the brain were also analyzed similarly. Specifically, proteins found to have 4-times expression relative to the expression in other tissues in the HPA data were extracted from the proteins detected in the plasma and serum extracellular vesicles, and those showing a strong correlation of expression in covariation analysis were further extracted. As examples, lymphoid-specific markers found in the plasma extracellular vesicles, skeletal muscle-specific markers and bone marrow-specific markers found in the serum extracellular vesicles, and liver tissue-specific extracellular vesicle markers found as many proteins in the plasma and serum extracellular vesicles will be mentioned. It is needless to say that, for tissues other than these tissues, tissue-specific proteins may be extracted by a similar method and be used as tissue-specific extracellular vesicle markers.

Among the proteins detected in the plasma extracellular vesicles, CD5L, GRAP2, GP1BA, CD3E, and CD3G were extracted as lymphoid-specific proteins based on the comparison with the HPA data. The correlation coefficients of expression among the proteins were obtained by the correlation analysis (Table 4). The expression of GRAP2, CD3E, and CD3G strongly covary with correlation coefficients of 0.8 or more, and it was suggested that they directly or indirectly interact with one another. Thus, GRAP2, CD3E, and CD3G are considered to be good markers for disease. Moreover, GRAP2, GP1BA, CD3E, and CD3G, among these, are considered to be also membrane proteins and may therefore be used to purify and/or concentrate lymphoid-specific extracellular vesicles.

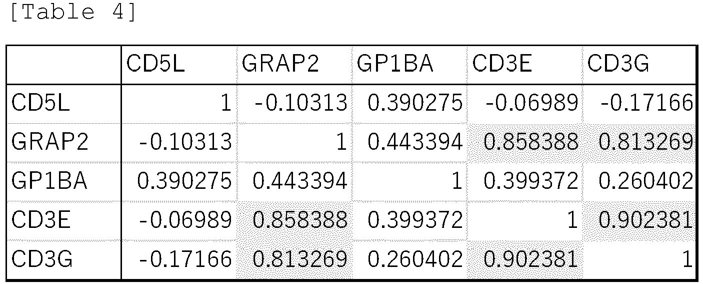

Next, the skeletal muscle-specific markers in the serum extracellular vesicles will be described. As extracellular vesicle markers found to have skeletal muscle-specific expression, XIRP2, STK25, ALDOA, PKM, PGM1, RAD23A, and CHMP1B were detected. The correlation analysis was conducted to obtain the correlation coefficients of expression among the proteins (Table 5). The expression of STK25, ALDOA, PKM, PGM1, and CHMP1B strongly covary with correlation coefficients of 0.8 or more, and it was suggested that they directly or indirectly interact with one another. Particularly, the 3 proteins ALDOA, PKM, and PGM1 correlate with one another, and are considered to be good markers for disease.

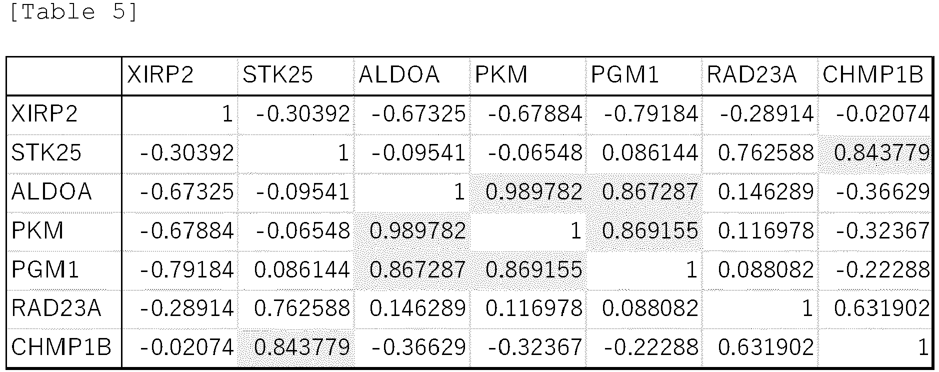

Next, the bone marrow-specific markers in the serum extracellular vesicles will be described. As extracellular vesicle markers found to have bone marrow-specific expression, HBD, SPTA1, MPO, HBB, HBA1, and RHAG were detected. The correlation coefficients of expression among the proteins were obtained by the correlation analysis (Table 6). The expression of HBD, SPTA1, HBB, and HBA1 strongly covary with correlation coefficients of 0.8 or more, and it was suggested that they directly or indirectly interact with one another. Particularly, the 3 proteins HBD, HBB, and HBA1 correlate with one another, and are considered to be good markers for disease. Moreover, RHAG is a membrane protein, and can be used as a target for EV purification.

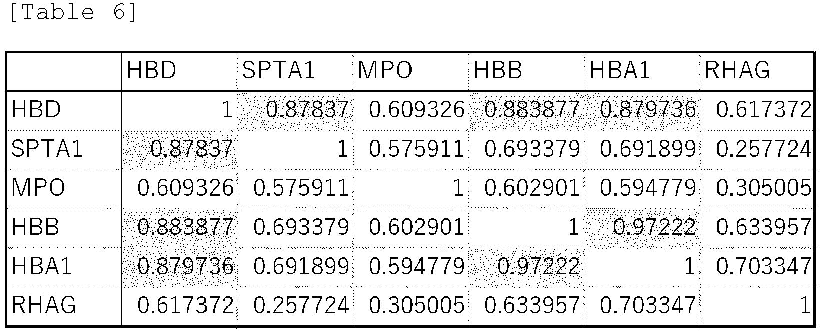

Also for the liver, liver-specific molecules present in the plasma extracellular vesicles and the serum extracellular vesicles were analyzed as described above. Among the proteins detected in the plasma extracellular vesicles, UGDH, ABCB11, ARG1, ADH4, MTHFD1, AKR1D1, CYP4F3, DCXR, SLC38A3, GRHPR, TFR2, and SLC27A5 were extracted. The correlation coefficients of expression among the proteins were obtained by the correlation analysis (Table 7). The expression of UGDH, ABCB11, ARG1, ADH4, MTHFD1, CYP4F3, DCXR, SLC38A3, GRHPR, and SLC27A5 strongly covary with correlation coefficients of 0.8 or more, and it was suggested that they directly or indirectly interact with one another. Moreover, ABCB11, CYP4F3, DCXR, SLC38A3, TFR2, and SLC27A5 are considered to be also membrane proteins and may therefore be used to purify and/or concentrate liver cell-specific extracellular vesicles.

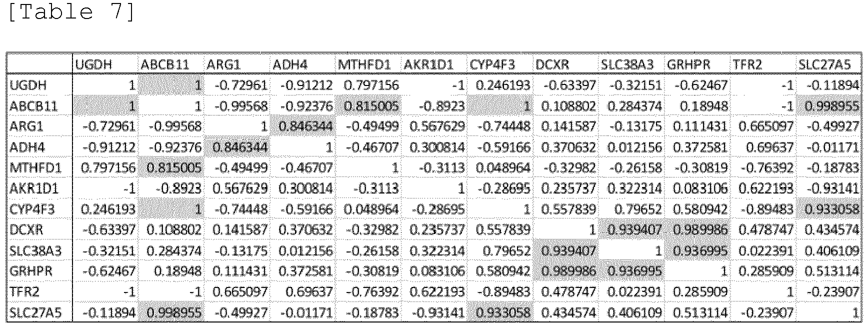

Next, the liver-specific proteins in the serum extracellular vesicles will be described. As extracellular vesicle markers found to have liver-specific expression, SLC27A2, ARG1, ADH4, MTHFD1, AKR1D1, CYP4F3, DCXR, RAB43, HGD, SLC38A3, GRHPR, TFR2, and SLC27A5 were extracted. The correlation coefficients of expression among the proteins were obtained by the correlation analysis (Table 8). The expression of SLC27A2, ARG1, ADH4, AKR1D1, CYP4F3, RAB43, HGD, SLC38A3, TFR2, and SLC27A5 strongly covary with correlation coefficients of 0.8 or more, and it was suggested that they directly or indirectly interact with one another. Moreover, SLC27A2, CYP4F3, DCXR, SLC38A3, TFR2, and SLC27A5 are considered to be also membrane proteins and may therefore be used to purify and/or concentrate liver-specific serum extracellular vesicles.

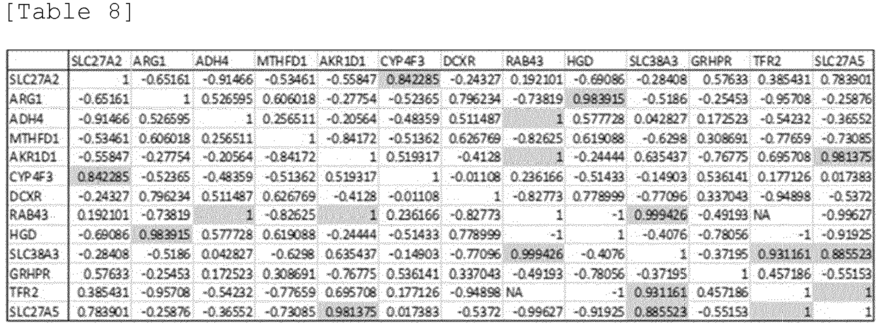

As described by way of example for brain, lymphocyte, skeletal muscle, bone marrow, and liver, tissue-specific extracellular vesicle markers can be obtained by analyzing proteins contained in extracellular vesicles and conducting a comparative analysis with information on expression in RNA sequence data. Furthermore, conducting the correlation analysis allows the selection of disease markers. Tissue-specific markers for extracellular vesicles are not only useful for concentrating tissue-specific extracellular vesicles, but also have a potential to be disease markers in themselves.

Next, tissue-specific proteins that are membrane proteins expressed on the extracellular vesicles and available for purifying tissue-specific extracellular vesicles were extracted. Among the proteins identified in the extracellular vesicles, proteins defined as tissue specific in HPA and not included in the plasma proteins in HPA, but existing as surface proteins or membrane proteins (cell membrane, endoplasmic reticulum, and Golgi apparatus) were extracted (Table 9). While no covariation analysis has been conducted therefor, the following markers are tissue-specific markers existing on the surface of extracellular vesicles and can suitably be used for purification. For example, after purification of extracellular vesicles, tissue-specific extracellular vesicles can be purified to a high degree of purity by using carriers such as beads to which antibodies or aptamers that bind to the tissue-specific markers listed in the table below are attached. A kit including a reagent containing an antibody, aptamer, or the like that binds to a tissue-specific marker is useful as a research reagent.

**[Table 9-1]**

| Tissue | Unipro ID | Gene Name | Description | plasma /serum | plasma | serum |
|---|---|---|---|---|---|---|
| Bone marrow | P02724 | GYPA | Glycophorin-A | * | | |
| | P05164 | MPO | Myeloperoxidase | * | | |
| | P08311 | CTSG | Cathepsin G | * | | |
| | Q02094 | RHAG | Ammonium transporter Rh type A | * | | |
| | Q5TGU0 | TSPO2 | Translocator protein 2 | | | |
| Brain | O60262 | GNG7 | Guanine nucleotide-binding protein G(I)/G(S)/G(O) subunit gamma- | * | | |
| | O75093 | SLIT1 | Slit homolog 1 protein | | * | |
| | P09104 | ENO2 | Gamma-enolase | * | | |
| | P14415 | ATP1B2 | Sodium/potassium-transporting ATPase subunit beta-2 | * | | |
| | P20336 | RAB3A | Ras-related protein Rab-3A | * | | |
| | P28223 | HTR2A | 5-hydroxytryptamine receptor 2A | | | * |
| | P31321 | PRKAR1B | cAMP-dependent protein kinase type I-beta regulatory subunit | * | | |
| | P51674 | GPM6A | Neuronal membrane glycoprotein M6-a | * | | |
| | P58743 | SLC26A5 | Prestin | | * | |
| | Q9NSK0; | KLC1 | Kinesin light chain 1 | * | | |
| | Q07866; | | | | | |
| | Q9H0B6 | | | | | |
| | Q13009 | TIAM1 | T-lymphoma invasion and metastasis-inducing protein 1 | * | | |
| | Q15334 | LLGL1 | Lethal(2) giant larvae protein homolog 1 | * | | |
| | Q16799 | RTN1 | Reticulon-1 | * | | |
| | Q8WWI5 | SLC44A1 | Choline transporter-like protein 1 | * | | |
| | Q93045 | STMN2 | Stathmin-2 | * | | |
| | Q96NW7 | LRRC7 | Leucine-rich repeat-containing protein 7 | * | | |
| | Q9BQI7 | PSD2 | PH and SEC7 domain-containing protein 2 | * | | |
| | Q9BSA4 | TTYH2 | Protein tweety homolog 2 | * | | |
| | Q9H115 | NAPB | Beta-soluble NSF attachment protein | * | | |
| | Q9UK28 | TMEM59L | Transmembrane protein 59-like | * | | |
| | Q9UQB3 | CTNND2 | Catenin delta-2 | * | | |
| | Q9Y2H9 | MAST1 | Microtubule-associated serine/threonine-protein kinase 1 | a | | |
| | Q9Y345 | SLC6A5 | Sodium- and chloride-dependent glycine transporter 2 | * | | |
| | Q9Y4B5 | MTCL1 | Microtubule cross-linking factor 1 | | | * |
| Breast | Q96J66 | ABCC11 | ATP-binding cassette sub-family C member 11 | | * | |
| Heart muscle | Q99959 | PKP2 | Plakophilin-2 | * | | |
| Intestine | 043451 | MGAM | Maltase-glucoamylase, intestinal | * | | |
| | P09327 | VIL1 | Villin-1 | | | * |
| | P19075 | TSPAN8 | Tetraspanin-8 | * | | |
| | Q01970 | PLCB3 | 1-phosphatidylinositol 4,5-bisphosphate phosphodiesterase beta-3 | * | | |
| | Q12864 | CDH17 | Cadherin-17 | | * | |
| | Q5MY95 | ENTPD8 | Ectonucleoside triphosphate diphosphohydrolase 8 | | * | |
| | Q99795 | GPA33 | Cell surface A33 antigen | * | | |
| | Q9BYE9 | CDHR2 | Cadherin-related family member 2 | | * | |
| | Q9ULC5 | ACSL5 | Long-chain-fatty-acid--CoA ligase 5 | * | | |

**[Table 9-2]**

| Tissue | Unipro ID | Gene Name | Description | plasma /serum | plasma | serum |
|---|---|---|---|---|---|---|
| Kidney | Q13113 | PDZK1IP1 | POZK1-interacting protein 1 | * | | |
| | Q6ZQN7 | SLCO4C1 | Solute carrier organic anion transporter family member 4C1 | * | | |
| | Q9NQA5 | TRPV5 | Transient receptor potential cation channel subfamily V member 5 | * | | |
| Liver | O14975 | SLC27A2 | Very long-chain acyl-CoA synthetase | | | * |
| | O95342 | ABCB11 | Bile salt export pump | | * | |
| | P21439 | ABCB4 | Phosphatidylcholine translocator ABCB4 | * | | |
| | Q08477 | CYP4F3 | Cytochrome P450 4F3 | * | | |
| | Q7Z4W1 | DCXR | L-xylulose reductase | * | | |
| | Q99624 | SLC38A3 | Sodium-coupled neutral amino acid transporter 3 | * | | |
| | Q9UP52 | TFR2 | Transferrin receptor protein 2 | * | | |
| | Q9Y2P5 | SLC27A5 | Bile acyl-CoA synthetase | * | | |
| Lung | P07988 | SFTPB | Pulmonary surfactant-associated protein 8 | * | | |
| | Q96PL1 | SCGB3A2 | Secretoglobin family 3A member 2 | | * | |
| Lymphoid | 060911 | CTSV | Cathepsin L2 | * | | |
| | P06126 | CD1A | T-cell surface glycoprotein COla | * | | |
| | P07359 | GP1BA | Platelet glycoprotein lb alpha chain | * | | |
| | P07766 | CD3E | T-cell surface glycoprotein CD3 epsilon chain | * | | |
| | P09693 | CD3G | T-cell surface glycoprotein CD3 gamma chain | * | | |
| | P20023 | CR2 | Complement receptor type 2 | * | | |
| | P16389; | KCNA3 | Potassium voltage-gated channel subfamily A member 3 | * | | |
| | Q09470; | | | | | |
| | P22001 | | | | | |
| | Q8NDX1 | PSD4 | PH and SEC7 domain-containing protein 4 | * | | |
| Pancreas | P35030 | PRSS3 | Trypsin-3 | * | | |
| | Q8WUX1 | SLC38A5 | Sodium-coupled neutral amino acid transporter 5 | * | | |
| Parathyroid | 095573 | ACSL3 | Long-chain-fatty-acid--CoA ligase 3 | * | | |
| | Q6YHK3 | CD109 | CD109 antigen | * | | |
| | Q9NRA2 | SLC17A5 | Sialin | * | | |
| | Q9UHI5 | SLC7A8 | Large neutral amino acids transporter small subunit 2 | * | | |
| Placenta | O60476 | MAN1A2 | Mannosyl-oligosaccharide 1,2-alpha-mannosidase IB | * | | |
| | P11166 | SLC2A1 | Solute carrier family 2, facilitated glucose transporter member 1 | * | | |
| | P51884 | LUM | Lumican | * | | |
| | Q9UIQ6 | LNPEP | Leucyl-cystinyl aminopeptidase | * | | |
| Retina | Q8TDN2 | KCNV2 | Potassium voltage-gated channel subfamily V member 2 | . | | |
| Salivary gland | P01036 | CST4 | Cystatin-S | | * | |
| | P02810 | PRH1 | Salivary acidic proline-rich phosphoprotein 1/2 | * | | |
| | P02814 | SMR3B | Submaxillary gland androgen-regulated protein 3B | | * | |
| | QSTAX7 | MUC7 | Mucin-7 | | * | |
| | Q96DA0 | ZG168 | Zymogen granule protein 16 homolog B | * | | |
| Skeletal muscle | A4UGR9 | XIRP2 | Xin actin-binding repeat-containing protein 2 | * | | |
| | Q96DD7 | SHISA4 | Protein shisa-4 | * | | |
| Skin | Q08554 | DSC1 | Desmocollin-1 | * | | |
| | Q15828 | CST6 | Cystatin-M | * | | |
| | Q53RT3 | ASPRV1 | Retroviral-like aspartic protease 1 | * | | |
| | Q81W75 | SERPINA12 | Serpin A12 | * | | |
| | QSTCV5 | WFDC5 | WAP four-disulfide core domain protein 5 | * | | |
| Testis | Q8TOB8 | SLC2A14 | Solute carrier family 2, facilitated glucose transporter member 14 | | * | |
| | Q96RT6 | CTAGE1 | cTAGE family member 2 | | | * |
| | Q9H8Y8 | GORASP2 | Golgi reassembly-stacking protein 2 | * | | |
| | Q9UI38 | PRSS50 | Probable threonine protease PRSS50 | | | * |
| Thyroid gland | Q86WI1 | PKHD1L1 | Fibrocystin-L | * | | |
| Tongue | P0DPA2 | VSIG8 | V-set and immunoglobulin domain-containing protein 8 | | * | |
| | P31025 | LCN1 | Lipocalin-1 | * | | |

By using an antibody or an aptamer that specifically binds to one of these proteins, tissue-specific extracellular vesicles can be purified. For example, by using an antibody or an aptamer that specifically binds to at least one of the brain tissue-specific markers on the surface of the membrane of extracellular vesicles described herein, brain tissue-specific extracellular vesicles can be purified. Furthermore, brain tissue-specific markers that covary have the high potential to be a diagnostic marker. Therefore, by analyzing the trend of these markers, it is possible to search for diagnostic markers with high sensitivity. With an antibody or an aptamer to be used for purification, a reagent that detects a diagnostic marker with high sensitivity may be used as a diagnosis kit. Furthermore, markers that change in the early stage of a neurodegenerative disease have the potential to be a new therapeutic target and can therefore also be applied to drug discovery. Enabling the purification of tissue-specific extracellular vesicles enables accurate diagnosis of disease.

### [Examples] Purification of brain tissue-specific extracellular vesicles

Brain tissue-specific extracellular vesicles were purified using an antibody to GPM6A, which is a brain tissue-specific marker. From serum samples obtained from healthy individuals, extracellular vesicles were purified with MagCapture Exosome Isolation Kit PS Ver.2, and then a biotinylated anti-GPM6A antibody (GeneTex, Inc.) or an antibody with the same isotype (Merck Millipore) was mixed by inversion at 4°C for 16 hours to cause a reaction. Streptavidin beads (Pierce) were added, and the beads were washed with PBS using a magnet stand. The extracellular vesicles bound to the biotinylated anti-GPM6A antibody and the isotype antibody were then collected using 2% sodium dodecyl sulfate. Respective extracellular vesicles were trypsinized, and LC-MS/MS analysis was then conducted. The result is shown in Table 10.

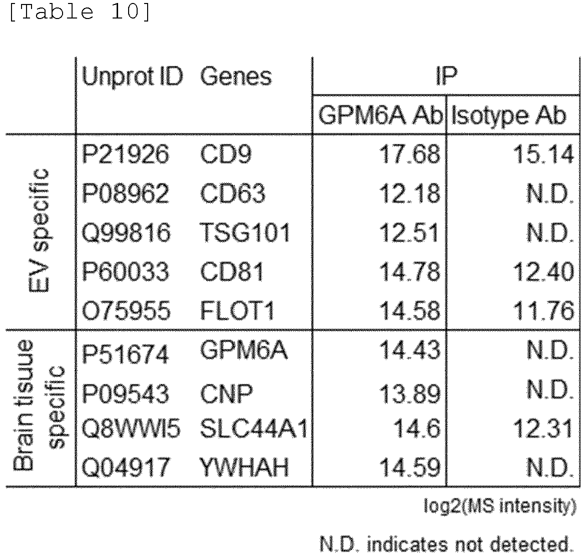

When extracellular vesicles were purified using the anti-GPM6A antibody, not only GPM6A used in immunoprecipitation, but also brain-specific proteins including CNP, SLC44A1, and YWHAH were markedly increased. It was found that brain-specific extracellular vesicles were concentrated, especially because GMP6A, CNP, and YWHAH were below the detection limit with the isotype antibody, while they were detected when the extracellular vesicles were purified using the anti-GPM6A antibody. Moreover, any of extracellular vesicle-specific proteins including CD9 was also concentrated, suggesting that brain-specific extracellular vesicles, among extracellular vesicles, were concentrated.

As shown in the foregoing, tissue-specific extracellular vesicles can be purified by using a tissue-specific extracellular vesicle marker. Here, the purification was attempted using one marker, but by using a plurality of markers, purification of more tissue-specific extracellular vesicles can be expected because tissue-specific extracellular vesicles do not constitute a homogeneous population. Purification of tissue-specific extracellular vesicles allows expectations for increased accuracy of diagnosis of disease and for the application to drug discovery.

## Claims

1. A method for purifying brain tissue-specific extracellular vesicles, comprising: purifying plasma extracellular vesicles or serum extracellular vesicles; and purifying brain tissue-specific extracellular vesicles using a specific reagent that binds to at least one marker selected, when using plasma extracellular vesicles, from GPM6A, LLGL1, CNP, MBP, and GNG7, which are detected in plasma extracellular vesicles, or selected, when using serum extracellular vesicles, from GDI1 and SLC44A1, which are detected in serum extracellular vesicles.

2. A brain tissue-specific marker, wherein the marker is at least one selected from GPM6A, YWHAH, LLGL1, CNP, STXBP1, MBP, and GNG7, which are detected in plasma extracellular vesicles, and GDI1, YWHAH, and SLC44A1, which are detected in serum extracellular vesicles.

3. A method for purifying liver tissue-specific extracellular vesicles, comprising, purifying plasma extracellular vesicles or serum extracellular vesicles; and using a specific reagent that binds to at least one marker selected, when using plasma extracellular vesicles, from ABCB11, CYP4F3, DCXR, SLC38A3, TFR2, SLC27A5, and ABCB4, which are detected in plasma extracellular vesicles, or selected, when using serum extracellular vesicles, from SLC27A2, CYP4F3, DCXR, SLC38A3, TFR2, SLC27A5, and ABCB4, which are detected in serum extracellular vesicles.

4. A liver tissue-specific marker, wherein the marker is at least one selected from ABCB11, CYP4F3, DCXR, SLC38A3, TFR2, SLC27A5, ABCB4, UGDH, ARG1, ADH4, MTHFD1, AKR1D1, and GRHPR, which are detected in plasma extracellular vesicles, and SLC27A2, CYP4F3, DCXR, SLC38A3, TFR2, SLC27A5, ABCB4, ARG1, ADH4, MTHFD1, AKR1D1, RAB43, HGD, and GRHPR, which are detected in serum extracellular vesicles.

5. A standard protein for plasma-derived extracellular vesicles, wherein the protein is at least one selected from NCK1 and ACTBL2.

6. A standard protein for serum-derived extracellular vesicles, wherein the protein is at least one selected from NCK1 and ARF6.

7. A method for selecting a standard protein for extracellular vesicles, comprising: analyzing extracellular vesicles from a plurality of healthy individuals; calculating coefficients of variation for detected proteins; and selecting a protein with a small coefficient of variation as the standard protein.

8. A method for selecting a tissue-specific marker in liquid biopsy, comprising: purifying extracellular vesicles from a body fluid; and, among proteins exhibiting tissue-specific expression in RNA sequence data showing information on human protein expression, selecting a protein detected in an extracellular vesicle fraction as a marker to be used for selection of tissue-specific extracellular vesicles.

9. The method for selecting a tissue-specific marker according to claim 8, wherein the proteins exhibiting tissue-specific expression in RNA sequence data are proteins found to exhibit at least 4 times change in the tissue of interest in comparison with other tissues.

10. The method for selecting a tissue-specific marker according to claim 8 or 9, wherein the RNA sequence data showing information on human protein expression is RNA sequence analysis data in Human Protein Atlas.

11. The method for selecting a tissue-specific marker according to any one of claims 8 to 10, wherein the method for purifying extracellular vesicles is affinity capture.

12. The method for selecting a tissue-specific marker according to any one of claims 8 to 11, wherein the body fluid is blood, and the extracellular vesicles are purified from plasma and/or serum.
